# EUROPEAN PATENT APPLICATION

(11) **EP 1 829 486 A1**
(43) Date of publication of application: **05.09.2007**
(21) Application number: 07101877.4
(22) Date of filing: 07.02.2007
(51) Int. Cl.: A61B 10/02, A61B 17/16, A61B 17/32

(54) **A surgical instrument for scraping and collecting bone particles**

(30) Priority: 02.03.2006 IT RE20060030
(71) Applicant: C.G.M. S.P.A., 42015 Correggio (Reggio Emilia) (IT)
(72) Inventor: Parmigiani, Corrado Saverio, 42015, Correggio (Reggio Emilia) (IT)
(74) Representative: Corradini, Corrado

(57) **Abstract**

The surgical instrument comprises a handle (10), which bears a blade (20) having a scraping edge (21), and a collection chamber (R) of shaved material. In the invention, the collection chamber (R) exhibits a mouth (S) for communication with an external environment, located in proximity of the scraping edge (21), for inlet of the shaved material. The mouth (S) is normally closed in order to prevent passage of material contained in the collection chamber (R). At least a portion (11 a) of the instrument is elastically deformable in order for the communication mouth (S) to be opened during a scraping operation by the scraping edge (21). In particular a wall (30) is exhibited, which, together with the handle (10) delimits the collection chamber (R); the wall (30) having an end portion (31) in proximity of the blade (20), which end portion (31) is separate and not constrained to the handle (10), the communication mouth (S) being located at a front end of the end portion (31). A portion (11a) of the handle is, at least in part, elastically deformable in order to distance the end portion (31) of the wall (30) from the handle (10) when the instrument is pressed, with the blade (20), against a bone, and to near the handle (10) when the instrument is not subject to mechanical solicitation.

## Description

The invention relates to reconstructive and regenerating removal methods of bone tissue during oral-maxillofacial, plastic, periodontal and implantological surgery, as well as plastic surgery techniques on bone.

In the past, instruments were developed for the removal of bone material in various zones of the skeletal structure, with the aim of obtaining granules and particles of suitable dimensions for biological regenerative tissue requirements.

These instruments have led to an increase in autologous bone harvesting and the treatment of the bone harvested, obtaining bone granules for filling bone deficits or for increasing skeleton structures.

In recent years the harvesting methodologies have been refined, thanks to the introduction onto the market of instruments for removing and collecting particles (flakes or chips) by filing bones, which instruments comprise a handle bearing a filing rasp located at the front end thereof, and a collection chamber of the removed material.

The above devices enable surface bone cortex removal by means of a special filing blade, with which the instrument is provided, which generates particles in the form of thin flakes or shavings which are directly collected in the collection chamber.

The high technological level of the filing blade means that with a light pressure an excellent and controllable shaving can be obtained. The collection of the filed bone particles is done by means of a passage (slit) located at the base of the blade, which conveys the particles internally of the collection chamber, defined by a special protected cell, which provides temporary quality maintenance space.

During the harvesting stage, the bone particles are mixed with blood to form a high-bone density concentrate, which is ideally suitable as a filler for grafting purposes.

An instrument, illustrated in WP 02/076307, by the same applicant, comprises a handle which bears a filing blade at a front end thereof, and possesses a collection chamber formed by the handle and a mobile wall associated to the handle. This instrument has a very slim transversal section so as to render its use in harvesting as minimally invasive as possible. A drawback of these instruments is that the collection chamber is in constant communication, even though through a relatively small opening mouth, with the outside environment and this can cause contamination of the biological material present therein.

An aim of the present invention is to improve this type of instrument, with the objective of realising a collection chamber which opens only when the blade of the instrument is pressed on the bone to scrape material from it, while it remains closed when the instrument is not in use for the above purpose.

A further aim of the invention is to prevent contact between the steel of the blade and the material collected in the collection chamber, as steel is not a biologically inert material (vis-a-vis the collected biological material) and could release contaminants into the said biological material.

A further aim of the invention is to realise an instrument which is able to remain in a stable position. not overturning when the mobile wall is separated from the handle in order for the material contained in the collection chamber to be accessed.

These and other aims are attained by the invention as it is characterised in the appended claims.

Thanks to the present invention, the collection chamber is in communication with the outside only during the bone material removal operation, while it is closed during the other stages and the biological material collected in the chamber is therefore in better conditions of sterility with respect to the known instruments in which the chamber is in constant communication, possibly through a small open mouth, with the external environment.

Further, when the chamber is closed, there is no contact between the collected material and the blade. The chamber can advantageously be completely made of a biologically inert plastic material, so as not to contaminate the biological material. The biological material is therefore in contact with the blade, the material of which is not entirely biologically inert, only when the blade is scraped on the bone.

The biological material can therefore be conserved in the collection chamber for a much longer time than is the case with known instruments.

The materials used to make the device are not of critical importance, except in the case of the blade which obviously must be made of steel. Preferably both the handle and the mobile wall are made of a synthetic resin suitable for medical use, such as for example polycarbonate or acrilonitryl/butadiene/styrene.

The invention is described herein below, with the aid of the accompanying figures of the drawings, which illustrate a non-exclusive preferred embodiment of the invention.
Figure 1 is a perspective three-quarter view from above of the instrument of the invention, in a closed configuration;
Figure 1A is the view of figure 1, where the instrument is in the open configuration;
Figure 1 B is an enlarged view of a detail of figure 1;
Figure 2 is a perspective three-quarter view from above of the instrument of the invention in a closed configuration, wherein the instrument is in a conventionally upturned position (i.e. with the rasping edge facing upwards);
Figure 2A is the view of figure 2, with the instrument in an open configuration;
Figure 2B is an enlarged detail of figure 2A;
Figure 3 is a lateral view of the instrument in a closed configuration;
Figure 4A is a plan view from above of the instrument in a closed configuration;
Figure 4B is a plan view from below of the instrument in a closed configuration;
Figure 5 is a front view of the front side of the instrument;
Figure 6 is a section according to the longitudinal axial plane VI-VI of figure 4A;
Figure 7A is an enlarged detail of figure 6, illustrating the front end of the instrument during a filing action on a bone;
Figure 7B is the same enlarged detail of figure 7A, with the instrument in an inactive stage;
Figure 8A is a section according to transversal plane A-A of figure 7A;
Figure 8B is a section according to transversal plane B-B of figure 7B;

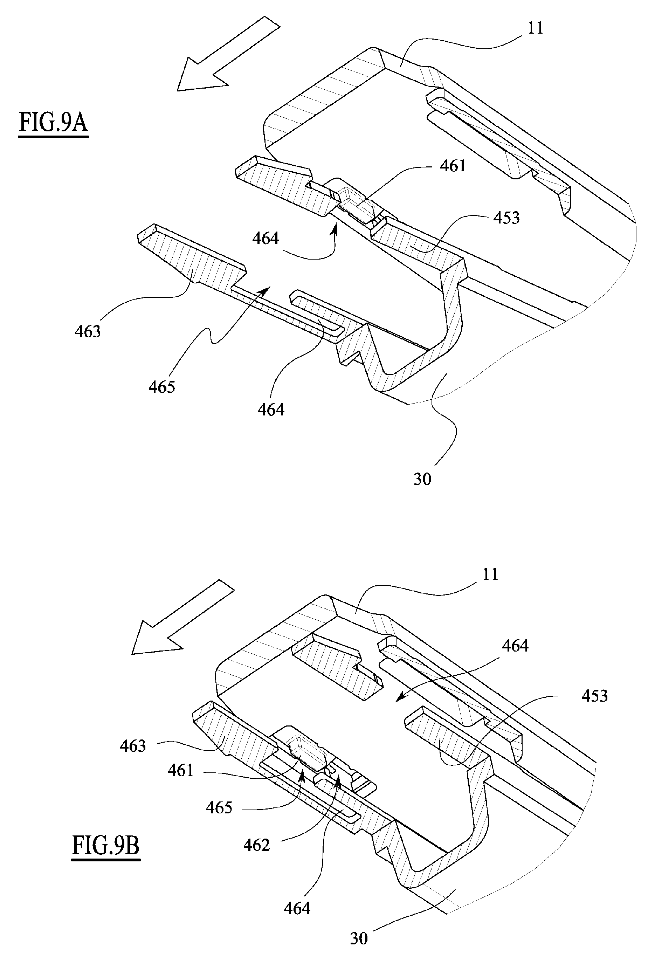

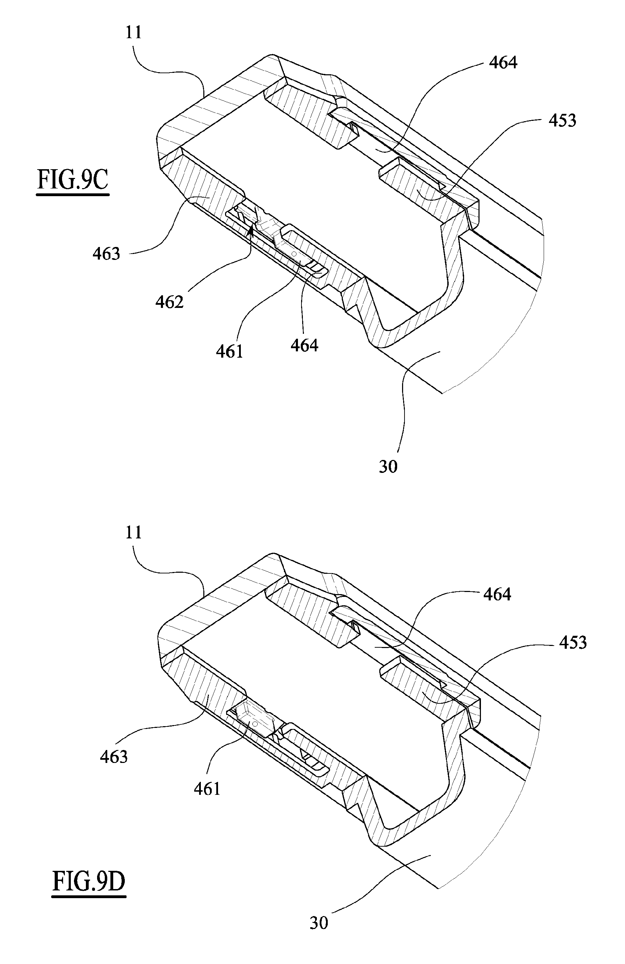

Figures 9A, 9B, 9C and 9D show a sequence of different positions of the portion of instrument, including means for constraining located between the handle and the mobile wall;
Figure 10 is a section according to plane X-X of figure 3, relating to the portion of instrument illustrated in figures 9A-9D.

The instrument of the invention comprises a handle 10 which bears a blade 20 having a rasping edge 21 which acts on the bone to scrape and remove biological material therefrom, and a collection chamber R of the scraped-off material.

In the illustrated embodiment of the figures, the instrument comprises a wall 30 which, together with the handle 10, delimits the collection chamber R.

The collection chamber R affords a mouth S for communication with the outside, located in proximity of the rasping edge 21, for the inlet of the removed material, which mouth S is normally closed in order to prevent the passage and especially the exit of the material contained in the collection chamber.

In a preferred embodiment, the handle is elongate, with a relatively small transversal section and a relatively extended longitudinal dimension, and the blade is fixed to the front end of the handle 10.

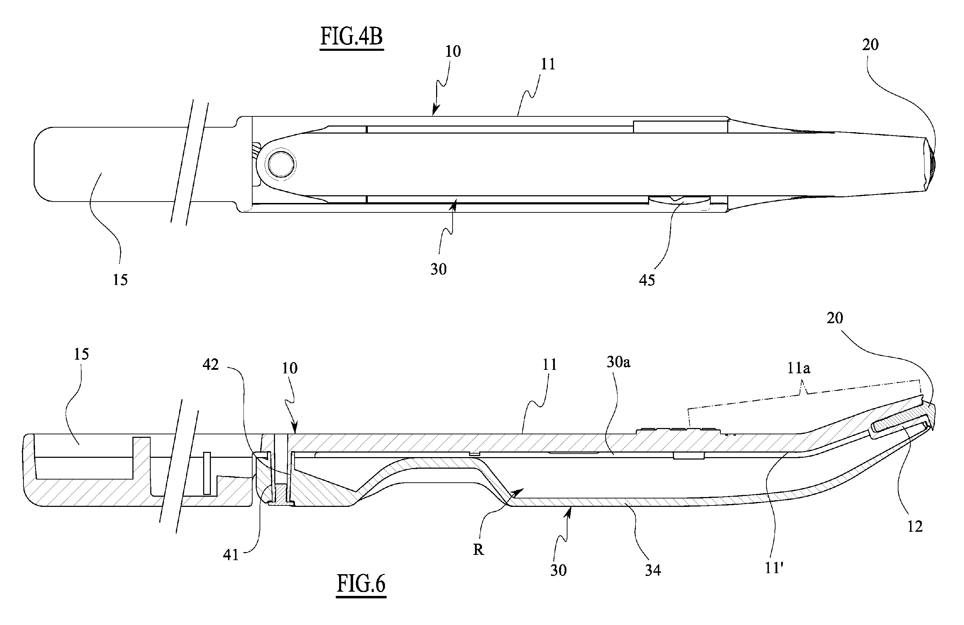

In more detail, the handle 10 comprises a back sector 15 and a front sector 11, which couples with the wall 30, at the front end of which the blade 20 is fixed. The blade 20 exhibits a tang 22 which is inserted, with the axis thereof parallel to the longitudinal axis of the instrument and the handle 10, internally of the front end 12 of the sector 11, and is surrounded and constrained solidly to the material of the sector 11. The blade 20 exhibits a substantially flat portion 23, facing downwards (in accordance with the conventional geometrical arrangement illustrated in figure 6) the edge of which flat portion 23 projects inferiorly beyond the front end 12 and defines the scraping edge 12. In particular, the flat portion 23 is more or less perpendicular to the axis of the tang 22.

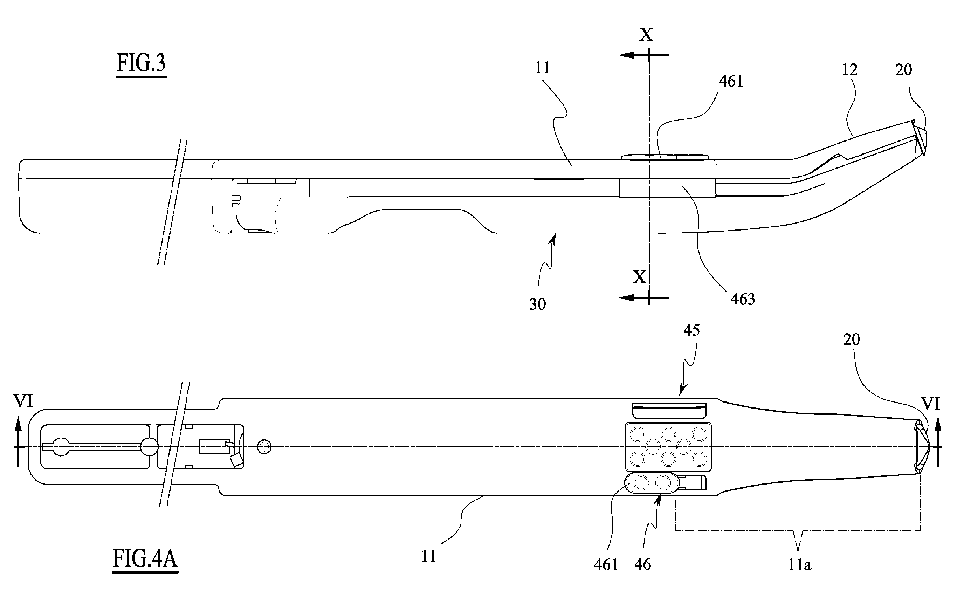

The wall 30 is arranged below (in accordance with the conventional geometrical arrangement illustrated in figure 3) the front sector 11 of the handle and is shaped such as to couple with the lower surface of the sector 11.

In detail, the lower surface 11' of the front sector 11 is substantially flat and defines a coupling surface with the wall 30. The wall has an upper edge 30a which geometrically marries with the corresponding lower surface 11' of the handle.

In the embodiment illustrated in the figures, the longitudinal axis of the instrument is not entirely straight but is subject to a deviation, with an acute angle, in the extreme front part thereof, in order to give rise to an ergonomic shape (figures 3 and 6). Consequently, the profile of the surface 11' and the profile of the upper edge 30a follow the same geometrical profile as the longitudinal axis.

Alternatively the longitudinal axis of the instrument can be entirely straight.

In a preferred embodiment, the wall 30 follows the elongate shape of the front sector 11 of the handle, in plan view, with a relatively small transversal section and a relatively large longitudinal dimension.

The collection chamber R is delimited only by the front sector 11 of the handle and by the wall 30.

The communication mouth S of the chamber R is located close to the portion 23 of the blade 20.

The front end 12 of the sector 11, in proximity of the portion 23, exhibits a tract 12a which bends downwards and adheres to the posterior surface 23' of the portion 23 of blade and exhibits an edge 13, facing downwards, which is inclined by an acute angle to the posterior surface 23' and is directed upwards and towards the inside of the chamber R.

At this edge 13, the extreme front portion of the wall 30 exhibits a front edge 33, parallel to the edge 13 and contacting the edge 13 when the instrument is not in use.

The mouth S of the chamber R is superiorly delimited by the lower edge 13 of the front end 12, and is inferiorly delimited by the front edge 33 of the wall 30.

The mouth S, when open, faces the posterior surface 23' of the scraping edge 21. In the embodiment illustrated in the figures, the scraping edge 21 has a convex curved profile which extends over a certain angle and consequently the mouth S and the relative edges 13 and 23 follow the arched profile.

When the mouth S is closed, the collection chamber R is entirely isolated from the external environment and the material collected internally thereof does not come into contact with the blade 20. All the parts delimiting the chamber R are made of an inert material, preferably an inert plastic material suitable for medical use.

The wall 30 is constrained to the handle 10, in particular to the front sector 11 thereof, by a pivot 41 constrained at the back end of the wall 30 and by two front constraints located side-by-side, 45 and 46, placed further forward at a same distance from the blade 20. The portion 31 of wall 30 located projectingly anteriorly of the two constraints 45 and 46 close to the blade 20 is free with respect to the handle 10, i.e. is separated and not constrained to the front sector 11 of the handle, and the communication mouth S is located at the front end of the front portion 31.

According to the invention, at least a portion of the instrument is elastically deformable in such a way as to realise the opening of the communication mouth S during the scraping operation of the scraping edge 21.

In particular, at least a portion of the handle 10 is elastically deformable in such a way as to distance the front portion 31 of the wall with respect to the front sector 11 of the handle when the instrument is pressed, with the blade 20 against the bone, and to near the front portion 31 when the instrument is not subject to mechanical stress.

In greater detail, the portion 11a of the front sector 11 in the zone going from the constraints 45 and 46 to the front end is elastically deformable.

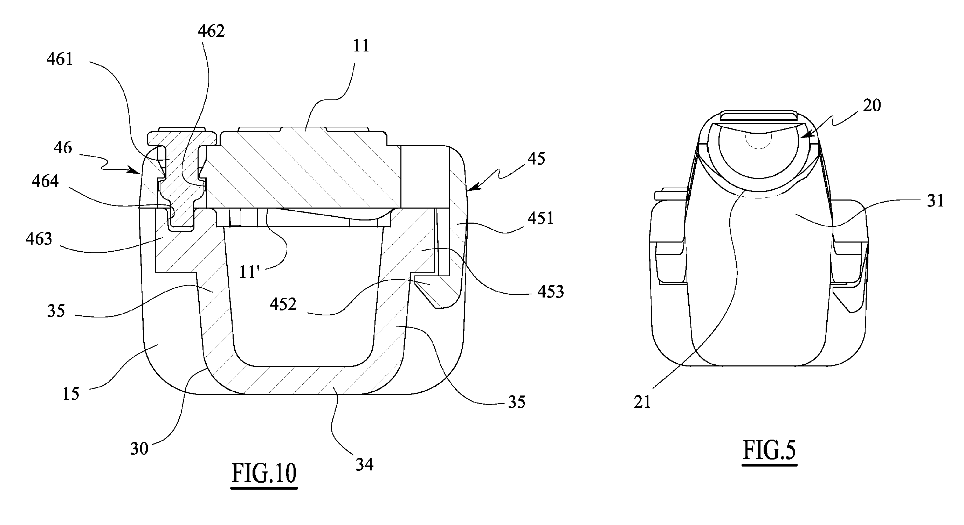

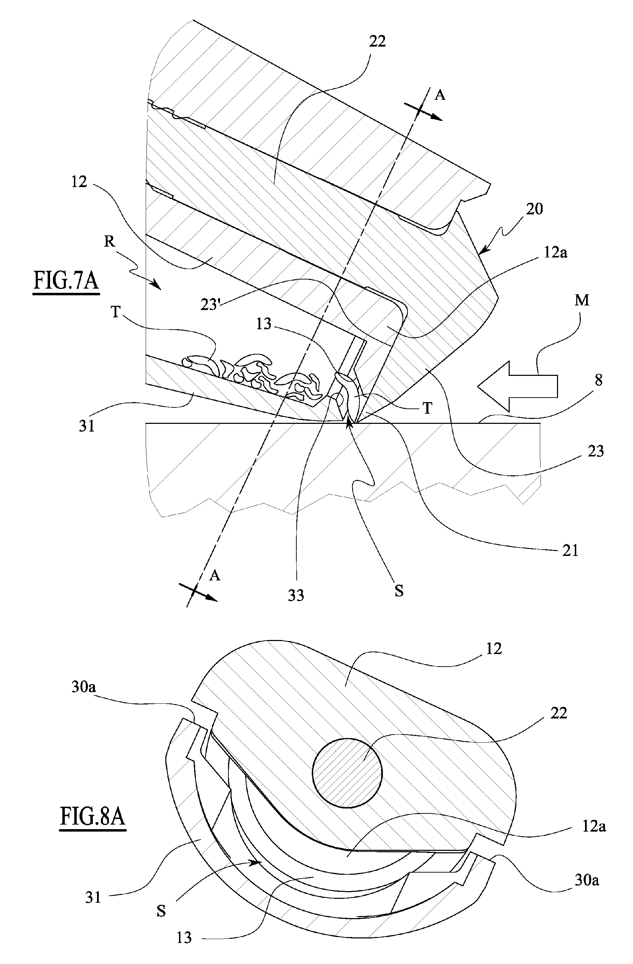

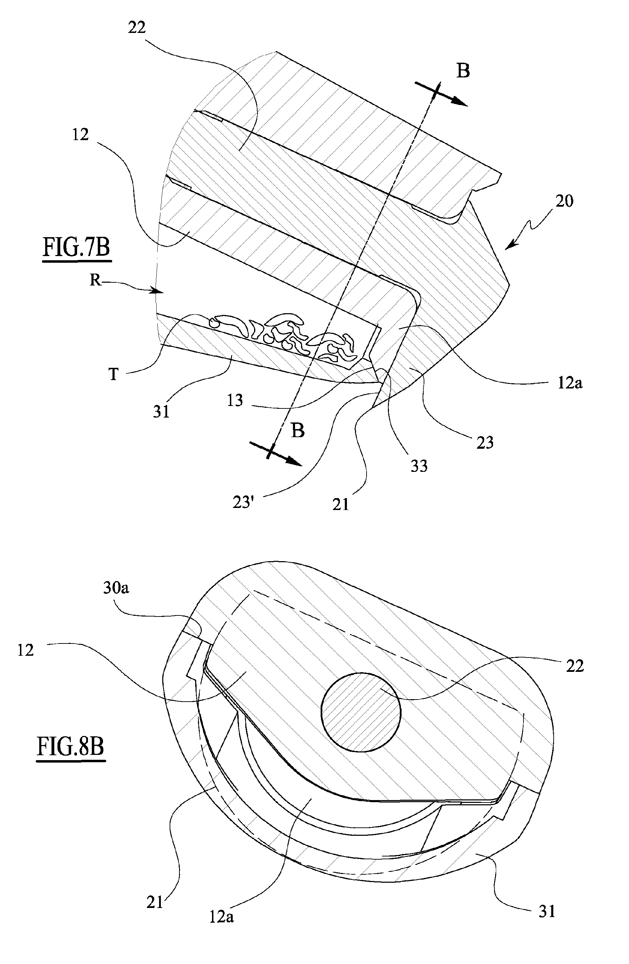

In use, the instrument is manipulated by gripping the handle 10, in particular the back sector 15 thereof; the front tract of the instrument, and in particular the front portion 11a of the handle, remains free from the hand's grip. When the scraping edge 21 is pressed downwards against the surface of the bone 8 (as illustrated in figure 7A), the portion 11a flexes elastically due to the posterior constraint produced by the pressure of the hand as it grips, and the constraint at the point caused by the fact that the blade 20 is against the bone 8, forming a slight arching, the concavity of which is facing away from the bone 8. The front portion 31 of the wall is not resting on the bone 8 and therefore does not deform, and rather oscillates downwards, distancing the front end thereof from the front end 12 of the handle, up until the front end is brought into contact with or almost with the bone 8. Consequently the edge 33 lowers and distances from the edge 13, which is stationary together with the blade 20, and thus the mouth S opens (figures 7A and 8A). During this stage, by pulling the instrument backwards (in the direction of the arrow M), while the edge 12 is pressed against the bone 8, the material scraped by the scraping blade 21 (schematically illustrated in figure 7A by shavings T) is pushed by the back surface 23' of the scraping blade 21 internally of the collection chamber R through the mouth S which is during this stage is open. The scraped shavings, pushed by the scraping edge 21, are guided by the edge 13 and 33 surfaces and pushed into the chamber R.

When the hand's action on the instrument, pushing the blade against the bone, stops, the mechanical solicitation on the portion 11 a of the handle also ceases and the handle returns to its normal configuration, nearing to the front portion 31 of the wall 30, bringing the edge 13 thereof into contact with the edge 33, thus closing the mouth S and isolating the chamber R from the external environment. This contact preferably occurs with a certain degree of pre-load, realised during the assembly stage of the components of the instrument, in order for the two edges 13 and 13 to come into contact with one another with a certain force, in order better to close the mouth S.

According to a characteristic of the invention, the wall 30 is constrained to the handle 10 with freedom to oscillate with respect to the handle 10, with rotation about an axis which is perpendicular or nearly perpendicular to the surface of reciprocal coupling.

In particular, the wall 30 has an arched transversal section, with concavity facing upwards, in order to form a channel-shaped receptacle which collects the material scraped by the blade 20 and which is superiorly closed by the front sector 11 of the handle 10. In more detail, the wall 30 possesses, in the general transversal section of the posterior and median parts thereof, a lower side 34 which is more or less horizontal and two sides 35 which are more or less vertical or inclined (see figure 10), which delimit a channel. In the more anterior part, as the wall 30 gets progressively nearer to the mouth S, the profile 30 thereof bows until it is arched (figures 8A and 8B).

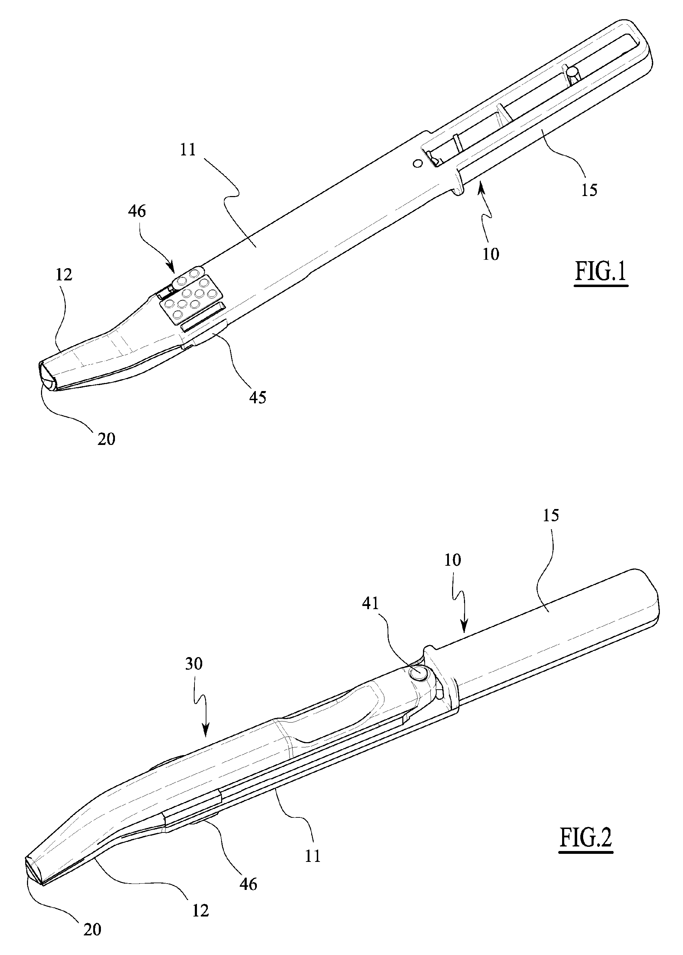

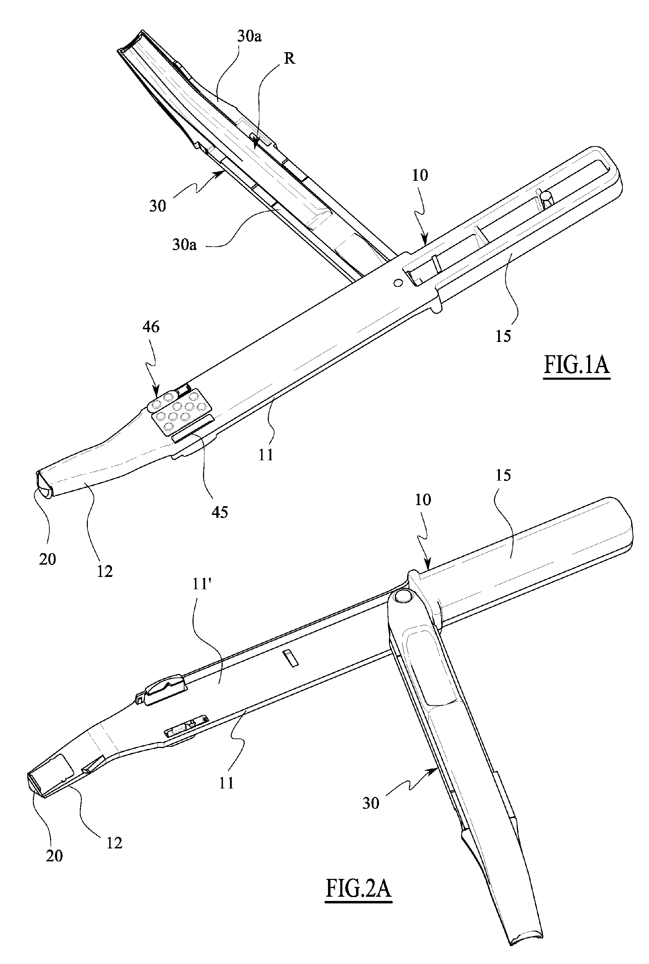

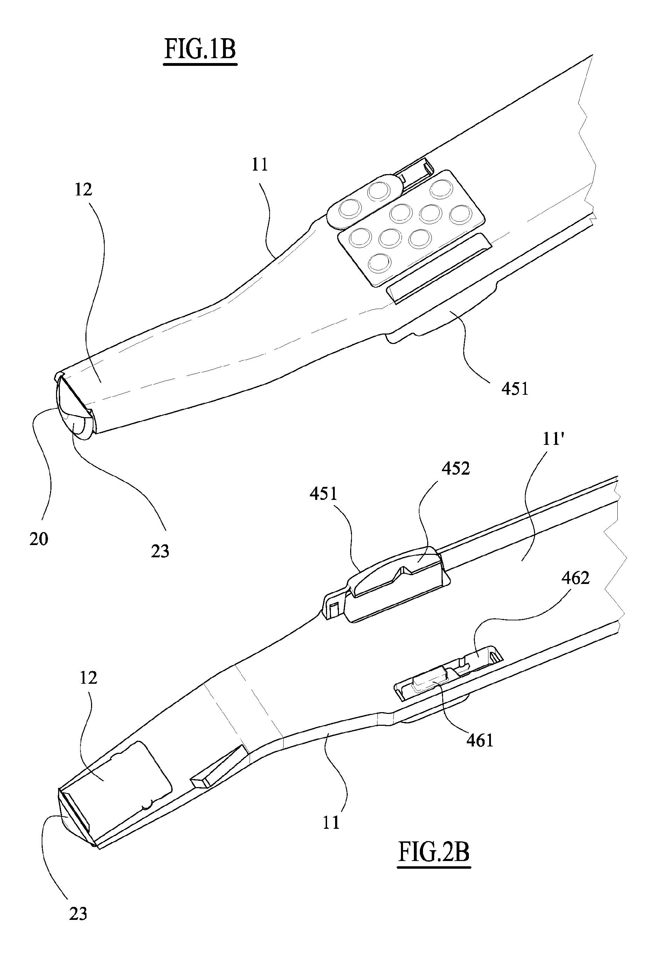

At the back end of the front sector 11 of the handle there is a pivot 41 projecting from the lower surface 11' in a perpendicular direction to the surface itself (i.e. the vertical direction in figure 6). The pivot 41 is inserted snugly in a hole 42 afforded at the back end of the wall 30, and is constrained therein in order not be able to exit the hole 42. Therefore, by means of the constraint 41, 42, the wall 30 can only oscillate with respect to the handle 10 with a rotation about the axis of the pivot 41. Thanks to this movement, the wall 30 can be separated and distanced from the handle 10 and make the material of the collection chamber R (contained in the wall 30) accessible from the outside, as shown in figure 1 A.

When the instrument is in the open configuration (figure 1A) it assumes a general T shape, which makes it very stable when placed on a plane and resistant to tilting, especially the position of the wall 30 where the biological material is collected; the operator can therefore leave the instrument on a plane without being forced to hold it with a hand in order to prevent its being tipped over, with a consequent loss of collected material.

The two front constraints 45 and 46 are located on substantially a same transversal plane and are different from one another.

The constraint 45 comprises a hook 451, made in a single body with the front sector 11 of the handle 10, projecting downwards and having a head 452 which forms an undercut with which the hook 451 engages with an upper edge 453 belonging to one of the lateral sides 35 of the wall 30, which projects laterally externally. The head 452 prevents downwards displacement of the edge 453 of the wall 30 with respect to the front sector 11 of the handle, and furthermore functions as a stop to the horizontal movement of the wall 30 towards the right.

The constraint 46 is located on the other lateral side 35 of the wall 30 and comprises a mobile body 461, slidable in a longitudinal direction parallel to the upper edge 463 of the lateral side 35. The body 461 is slidable within a longitudinal channel 462 afforded in the front sector 11, and is engaged in the channel 462 by means of undercuts which constrain it, enable only a sliding movement in a longitudinal direction. By means of other undercut elements between the body 461 and a channel 464 afforded in the edge 463, the body 461 is constrained in order that it cannot distance from the front sector 11 of the handle.

A passage opening 454 is afforded in the edge 453, which allows passage of the mobile body 461 through the edge itself, in order to enable coupling and decoupling between the wall 30 and the front sector 11 of the handle 10 following the rotation movement about the axis of the pivot 41 (see figures from 9A to 9D). Further, the channel 464 has an internal lateral opening which also allows passage of the mobile body 461.

## Claims

1. A surgical instrument for scraping and collecting bone particles, comprising a handle (10), which bears a blade (20) having a scraping edge (21), and a collection chamber (R) of shaved material, **characterised in that**:
the collection chamber (R) exhibits a mouth (S) for communication with an external environment, located in proximity of the scraping edge (21), for inlet of the shaved material, which mouth (S) is normally closed in order to prevent passage of material contained in the collection chamber (R);
at least a portion (11 a) of the instrument being elastically deformable in order for the communication mouth (S) to be opened during a scraping operation by the scraping edge (21).

2. The instrument of claim 1, **characterised in that** it comprises a wall (30) which together with the handle (10) delimits the collection chamber (R); the wall (30) having an end portion (31) in proximity of the blade (20), which end portion (31) is separate and not constrained to the handle (10), the communication mouth (S) being located at a front end of the end portion (31).

3. The instrument of claim 2, **characterised in that** a portion (11 a) of the handle is at least in part elastically deformable in order to distance the end portion (31) of the wall (30) from the handle (10) when the instrument is pressed, with the blade (20), against the bone, and to near the handle (10) when the instrument is not subject to mechanical solicitation.

4. The instrument of claim 3, **characterised in that** the handle (10) bears the blade at a front end thereof (12), and the front portion (11 a) is the elastically deformable portion, the communication mouth (S) being delimited on a side thereof by an end edge (13) of the handle (10) and on the other side thereof by an end edge (33) of the wall (30).

5. The instrument of claim 3, **characterised in that** the wall (30) is constrained to the handle (10), by means of two front constraints (45 and 46) located side-by-side, the portion of wall (30) located projectingly anteriorly of the two constraints (45 and 46) being free with respect to the handle (10) and defining the elastically deformable portion (11 a).

6. The instrument of claim 2, **characterised in that** the wall (30) has an upper edge which geometrically marries with a corresponding coupling surface (11') of the handle, the wall (30) being constrained to the handle (10) with freedom to oscillate with respect thereto, with a rotation about a perpendicular axis to the coupling surface (11').
